**Europäisches Patentamt**

**European Patent Office**

**Office européen des brevets**

(19)

(11) Veröffentlichungsnummer: **0 151 755**
**B1**

(12) # EUROPÄISCHE PATENTSCHRIFT

(45) Veröffentlichungstag der Patentschrift:
18.03.87

(21) Anmeldenummer: 84115398.4

(22) Anmeldetag: **13.12.84**

(51) Int. Cl.⁴: **C 07 C 43/13,** C 07 C 41/09,
**C 08 G 65/34**

(54) **Verfahren zur Herstellung von Polyglycerinen.**

(30) Priorität: **21.12.83 DE 3346097**

(43) Veröffentlichungstag der Anmeldung:
**21.08.85 Patentblatt 85/34**

(45) Bekanntmachung des Hinweises auf die Patenterteilung:
**18.03.87 Patentblatt 87/12**

(84) Benannte Vertragsstaaten:
**BE CH DE FR GB IT LI NL**

(56) Entgegenhaltungen:
**US-A-3 637 774**
**US-A-3 968 169**

(73) Patentinhaber: **HOECHST AKTIENGESELLSCHAFT,
Postfach 80 03 20, D-6230 Frankfurt am Main 80
(DE)**

(72) Erfinder: **Stühler, Herbert, Dr., Hochfellnstrasse
12, D-8269 Burgkirchen (DE)**

**Beschreibung**

Die Erfindung betrifft ein Verfahren zur Herstellung von Polyglycerinen mit guter Farbqualität und Geruchsneutralität.

Polyglycerine sind bekanntlich Kondensationsprodukte (Polymerisationsprodukte), die aus Glycerin unter intermolekularer Abspaltung von Wasser entstehen und den weiten Bereich vom Diglycerin (mit 2 Glycerinradikalen oder Glycerineinheiten) bis beispielsweise zum Triacontaglycerin (mit 30 Glycerineinheiten) umfassen. Im Rahmen der vorliegenden Erfindung geht es im wesentlichen um jene Polyglycerine, die aus 2 bis 25, vorzugsweise 2 bis 15 Glycerineinheiten bestehen.

Polyglycerine sind wertvolle und vielseitig verwendbare Verbindungen, beispielsweise zur Herstellung von oberflächenaktiven Substanzen, wie Estern oder oxalkylierten Estern. Diese Folgeprodukte werden vorteilhaft als Emulgatoren im Nahrungsmittelsektor, bei der Formulierung von kosmetischen und pharmazeutischen Präparaten und im Waschmittelsektor sowie als Gleitmittel bei der Verarbeitung von Kunststoffen eingesetzt. Aus diesen Verwendungszwecken resultiert die Forderung nach einer guten Farbqualität und Geruchsneutralität. Es sollte demnach ein Verfahren zur Verfügung stehen, mit dem Polyglycerine farblos oder nahezu farblos und gleichzeitig möglichst geruchsneutral hergestellt werden können.

Es hat nicht an Versuchen gefehlt, dieses Ziel zu erreichen. Ausgehend von dem älteren Stand der Technik, wonach Polyglycerine durch Umsetzung von Glycerin in Gegenwart von alkalischen oder von sauren Katalysatoren unter Entfernung des bei der Umsetzung entstehenden Reaktionswassers erhalten werden können, ist in neuerer Zeit verstärkt versucht worden, diese Reaktion mit Hilfe von speziellen Katalysatoren und/oder mit Hilfe einer speziellen Reaktionsführung auf möglichst wenig gefärbte Polyglycerine zu steuern.

So wird in der US-Patentschrift 3 637 774 zur Herstellung von Polyglycerin mit guter Farbqualität empfohlen, Glycerin in Gegenwart von alkalischen Katalysatoren wie Natriumhydroxid, Kaliumhydroxid, Alkalimetallalkoholate, Natriumacetat, Metalloxide und dergleichen, und in Abwesenheit von Wasser bei einer Temperatur von 100 bis 300 °C unter kontinuierlicher Austragung des Reaktionswassers umzusetzen (zu kondensieren, zu polymerisieren), das Reaktionsprodukt schnell abzukühlen, in Wasser aufzunehmen und mit einem Bleichmittel bei einer Temperatur unterhalb von 100 °C und oberhalb Raumtemperatur zu behandeln. Dieses Verfahren läßt insbesondere dadurch zu wünschen übrig, daß ohne die Durchführung der Bleichung relativ stark gefärbte und stark riechende (stechender Geruch nach Acrolein) Polyglycerine erhalten werden.

Bei dem in der US-Patentschrift 3 968 169 beschriebenen Verfahren zur Herstellung von Polyglycerin wird (a) Glycerin unter bestimmten Temperatur- und Druckbedingungen in Gegenwart eines Katalysatorsystems aus Schwefelsäure und einem Glycerinester auf eine Temperatur von 110 bis 180 °C erhitzt, bis etwa 25 bis 75 % des Glycerins polymerisiert sind, (b) die Schwefelsäure durch Zusatz einer im wesentlichen stöchiometrischen Menge eines Neutralisationsmittels inaktiviert und (c) das nicht-umgesetzte Glycerin durch Destillation entfernt. Dieses Verfahren ist, wie ohne weiteres ersichtlich, wegen der erforderlichen Einhaltung mehrerer spezifischer Reaktionsbedingungen relativ kompliziert. Darüberhinaus resultieren Produkte mit einer relativ schlechten Farbqualität.

Die Aufgabe der vorliegenden Erfindung besteht demnach darin, ein Verfahren zur Herstellung von Polyglycerinen durch Kondensation von Glycerin zur Verfügung zu stellen, das direkt zu geruchsneutralen Produkten mit guter Farbqualität führt. Das neue Verfahren soll ferner an keine komplizierte Reaktionsführung gebunden sein, es soll sich also um ein im Prinzip einfaches Verfahren handeln.

Es wurde überraschenderweise gefunden, daß die in Rede stehende Kondensation dann zu Polyglycerinen mit guter Farbqualität und Geruchsneutralität führt, wenn als Katalysator reduzierend wirkender Phosphor und Alkalimetall in einer bestimmten Menge und in einem bestimmten Verhältnis zueinander vorliegen. Mit diesem Katalysatorsystem werden aus Glycerin im Rahmen einer einfachen Reaktionsführung und ohne eine Bleichbehandlung durchführen zu müssen, Polyglycerine mit einer unerwartet hohen Farbqualität und Geruchsneutralität erhalten.

Das erfindungsgemäße Verfahren zur Herstellung von Polyglycerinen, bei dem Glycerin in Gegenwart von Katalysatoren bei einer Temperatur von 190 bis 250 °C unter Entfernung des Reaktionswassers umgesetzt wird, ist dadurch gekennzeichnet, daß als Katalysator Phosphor und Alkalimetall enthaltende Verbindungen eingesetzt werden, so daß 0,005 bis 1 Gew.-%, bezogen auf das Gewicht von Glycerin, Phosphor vorliegt und das Molverhältnis von Alkalimetall zu Phosphor 1 bis 12 : 1 beträgt, wobei die phosphor- und alkalimetallenthaltenden Verbindungen ausgewählt sind aus der Gruppe bestehend aus reduzierenden Phosphorsäuren, Alkalisalzen von reduzierenden Phosphorsäuren, Alkalihydroxiden, Alkalicarbonaten, Alkalibicarbonaten, Alkalialkoholaten und Alkalioxiden.

Liegt der Anteil an Phosphor unter 0,005 Gew.-%, bezogen auf eingesetztes Glycerin, so läuft die Kondensationsreaktion zu Polyglycerin nur noch sehr langsam ab. Anteile über 1 Gew.-% bringen keine wesentliche zusätzliche Wirkung mehr. Bei einem Molverhältnis von Alkalimetall : Phosphor von > als 12 : 1 sind zwar relativ kurze Reaktionszeiten möglich, die Jodfarbzahl des erhaltenen Polyglycerins steigt jedoch stark an. Beträgt dieses Verhältnis weniger als 1 : 1, so geht die Umsetzung des Glycerins auch bei hohen Temperaturen nur noch sehr langsam vor sich.

Die phosphor- und alkalimetallenthaltenden Verbindungen werden vorzugsweise in einer solchen Menge eingesetzt, daß 0,02 bis 0,3 Gew.-% (bezogen auf das Gewicht des eingesetzten Glycerins) Phosphor zugegen sind und das Molverhältnis von Alkalimetall : Phosphor 2 bis 5 : 1 beträgt.

Zur Erreichung der angegebenen Menge an Phosphor und Alkalimetall kann so vorgegangen werden, daß

2

von den genannten Verbindungen solche eingesetzt werden, die sowohl Phosphor als auch Alkalimetall enthalten, oder daß Phosphorverbindungen (die kein Alkalimetall enthalten) und Alkalimetallverbindungen (die keinen Phosphor enthalten) genommen werden. Es können auch phosphor- und alkalimetallenthaltende Verbindungen, beispielsweise $Na_2HPO_3$ oder $NaH_2PO_2$, und (reine) Alkalimetallverbindungen, beispielsweise NaOH oder $Na_2CO_3$, eingesetzt werden. Wichtig ist lediglich, daß die angegebene Phosphor- und Alkalimetallmenge insgesamt vorliegt.

Reduzierende Phosphorsäuren sind bekanntlich solche mit der Oxidationszahl (Oxidationsstufe) $+1$, $+3$ und $+4$. Vertreter davon sind die Unterphosphorige Säure, $H_3PO_2$ (ihre Salze heißen Hypophosphite), Phosphorige Säure, $H_3PO_3$ (ihre Salze heißen Phosphite), Diphosphorige Säure, $H_4P_2O_5$ (ihre Salze heißen Diphosphite) und die Unter-diphosphorsäure, $H_4P_2O_6$ (ihre Salze heißen Hypodiphosphate).

Als phosphor- und alkalimetallenthaltende Verbindungen werden vorzugsweise die folgenden eingesetzt (einzeln oder in Mischungen): Unterphosphorige Säure, Phosphorige Säure, Alkalisalze der Unterphosphorigen Säure, Alkalisalze der Phosphorigen Säure, Alkalihydroxide, Alkalicarbonate, Alkalibicarbonate, Alkalialkoholate, vorzugsweise Methylate, Ethylate und Propylate, Alkalioxide oder Mischungen davon. Besonders geeignet sind Unterphosphorige Säure, Phosphorige Säure oder Mischungen davon als Phosphorverbindung (Katalysatorkomponente a) und Alkalihydroxide, Alkalicarbonate, Alkalibicarbonate, Alkalialkoholate, Alkalioxide oder Mischungen davon als Alkalimetallverbindung (Katalysatorkomponente b), wobei von den Alkalimetallverbindungen die Hydroxide, Carbonate und die Bicarbonate bevorzugt sind.

Alkalimetall bedeutet vorzugsweise Kalium oder Natrium.

Als Glycerin wird zweckmäßigerweise ein möglichst reines Produkt eingesetzt, das ist ein sogenanntes hochwertiges Glycerin, z. B. das DAB8 Glycerin (DAB8 = Deutsches Arzneimittelbuch 8. Auflage).

Die Kondensation von Glycerin unter Verwendung des erfindungsgemäßen Katalysatorsystems wird bei einer Temperatur von 190 bis 250 °C, vorzugsweise 200 bis 230 °C, durchgeführt. Bei einer Temperatur von < 190 °C verläuft die Kondensationsreaktion im Hinblick auf eine praktische Anwendung zu langsam und bei mehr als 250 °C nimmt die Farbqualität des Kondensationsproduktes deutlich ab. Während der Kondensation wird vorzugsweise eine Inertgasatmosphäre gehalten. Dazu ist es zweckmäßig, sowohl während des Aufheizens als auch während der Kondensation zu rühren und ein Inertgas wie Stickstoff, Kohlendioxid und dergleichen über oder durch die Reaktionsmischung zu leiten. Der Inertgasstrom beträgt im allgemeinen 0,5 bis 20 Liter, vorzugsweise 1 bis 15 Liter, Gas pro Stunde und pro kg Reaktionsmischung. Die erfindungsgemäße Umsetzung wird in der Regel bei Atmosphärendruck oder geringem Unterdruck, das ist ein Druck von vorzugsweise 2.000 bis 4.000 Pa (Wasserstrahlvakuum) durchgeführt, wobei gegebenenfalls bereits vorhandenes Wasser und das entstehende Reaktionswasser destillativ ausgetragen werden. Durch Messung der Menge des Reaktionswassers und/oder durch Bestimmung der Hydroxylzahl, des Brechungsindex, des Molekulargewichts und/oder der Viskosität des Reaktionsproduktes kann der Verlauf der Umsetzung verfolgt und auf den gewünschten Grad der Glycerinkondensation gesteuert werden. Zur Beendigung der Umsetzung und zur Überführung des Reaktionsproduktes auf etwa Raumtemperatur wird dieses zweckmäßigerweise möglichst rasch abgekühlt.

Die Reaktionszeit hängt insbesondere von der Reaktionstemperatur und der Menge an Phosphor und Alkalimetall sowie dem Verhältnis der beiden zueinander ab. Sie ist relativ kurz bei einer hohen Reaktionstemperatur, einer hoher Katalysatormenge und einem hohen Verhältnis von Alkalimetall zu Phosphor.

Polyglycerine, das Reaktionsprodukt des erfindungsgemäßen Verfahrens, stellen bekanntlich eine bei Raumtemperatur (je nach Dehydratisierungsgrad, Kondensationsgrad) mehr oder weniger viskose Flüssigkeit dar. Das Reaktionsprodukt besteht in der Regel - abgesehen von gegebenenfalls nicht umgesetztem Glycerin - aus einer Mischung aus Polyglycerinen beginnend mit dem Diglycerin. Nachdem die gebräuchlichsten Polyglycerine die homologe Reihe mit Schwerpunkt Diglycerin bis Pentadecaglycerin umfassen, wird in der Regel ein derartiges Reaktionsprodukt angestrebt. Sollten die erhaltenen Polyglycerine von Katalysator und vom nicht umgesetzten Glycerin befreit werden, so kann die Abtrennung des Katalysators beispielsweise durch Behandlung des Reaktionsproduktes mit einem basischen und einem sauren Ionentauscher und die Abtrennung des Glycerins durch Destillation in einfacher Weise erreicht werden.

Das erfindungsgemäße Verfahren ist einfach in seiner Durchführung, da es keine besonderen Maßnahmen erfordert. Die Polyglycerine werden in einer unerwartet hohen Farbqualität und Geruchsneutralität erhalten. Sollte im Hinblick auf eine ganz bestimmte Verwendung eine außerordentliche Farbqualität erwünscht sein, so kann dies dadurch erreicht werden, daß die Produkte mit den hierfür üblichen Methoden, wie Bleichung mit Aktivkohle oder Wasserstoffperoxid und/oder Säulenchromatografie, behandelt werden.

Die Erfindung wird nun an Beispielen noch näher erläutert.

**Beispiel 1**

In einem mit Rührer, Thermometer, Gaseinleitungsrohr und Rückflußkühler mit Wasserabscheider ausgestattetem Reaktionsgefäß wurden vorgelegt 1.000 g Glycerin 98 gew.-%ig wäßrig (das sind 980 g 100 gew.-%ig), 0,26 g Unterphosphorige Säure 50 gew.-%ig wäßrig (das sind 0,13 g 100 gew.-%ig) und 0,32 g Natriumhydroxid 100 gew.-%ig (die 0,13 g Unterphosphorige Säure entsprechen 0,006 Gew.-% Phosphor, bezogen auf die 980 g Glycerin. Die 0,13 g Unterphosphorige Säure entsprechen 0,002 Mol Phosphor und die 0,32 g Natriumhydroxid entsprechen 0,008 Mol Natrium. Das Molverhältnis von Natrium zu Phosphor ist also 4 : 1). Es wurde bei Atmosphärendruck unter Rühren und Durchleiten von Stickstoff (ca. 5 l/Stunde) auf 250 °C erhitzt, wobei während des Erhitzens bereits Wasser abdestillierte. Nach Erreichen der Reaktionstemperatur von 250 °C wurde 72 Stunden lang bei dieser Temperatur und bei Atmosphärendruck und weiterem Rühren, Durchleiten des Stickstoffstromes und Abdestillieren von Wasser gehalten. Nach dieser Zeit hatte das Reaktionsprodukt eine Hydroxylzahl (OH-Zahl) von 1.360. Nun wurde auf Raumtemperatur abgekühlt.

Das Reaktionsprodukt (gemäß OH-Zahl Diglycerin) war eine klare und nahezu farblose Flüssigkeit. Ihre Iodfarbzahl war < 1 (vergleiche nachstehende Tabelle).

### Beispiele 2 bis 12

Die Beispiele 2 bis 12 wurden analog dem Beispiel 1 durchgeführt. Die zur Reaktion des Glycerins jeweils eingesetzten Phosphorverbindungen und Alkalimetallverbindungen, die Menge an diesen Verbindungen, das Molverhältnis von Alkalimetall zu Phosphor, die Reaktionsbedingungen sowie Hydroxylzahl und die Iodfarbzahl der erhaltenen Polyglycerine sind in der nachstehenden Tabelle zusammengefaßt.

### Vergleichsbeispiele 1 und 2

Die Vergleichsbeispiele 1 und 2 wurden analog dem Beispiel 1 durchgeführt mit der Ausnahme, daß als Katalysator nur Natriumhydroxid (Vergleichsbeispiel 1) und nur Unterphosphorige Säure (Vergleichsbeispiel 2) eingesetzt wurde. Die Menge an Natriumhydroxid 100 gew.-%ig betrug 1,6 Gew.-% und die Menge an Unterphosphoriger Säure 100 gew.-%ig 0,5 Gew.-%, bezogen auf das eingesetzte Glycerin.

Wie die Vergleichsbeispiele zeigen, sind die in beiden Fällen erhaltenen Polyglycerine braun gefärbt, vergleiche nachstehende Tabelle.

Die Iodfarbzahl, die bekanntlich zur Kennzeichnung der Farbe von Flüssigkeiten dient, wurde nach DIN (Deutsche Industrienorm) 6162 bestimmt. Iodfarbzahlen von kleiner 1 (< 1) bis 5 resultieren aus nahezu farblos bis schwach gelb gefärbten Produkten.

Die Hydroxylzahl, die bekanntlich zur Ermittlung des Gehaltes an Hydroxylgruppen dient, wurde nach der allgemein üblichen Methode gemäß DIN 53 240 bestimmt.

Im folgenden sei der Zusammenhang zwischen der Hydroxylzahl und dem Polymerisationsgrad des Polyglycerins näher veranschaulicht (vergleiche die eingangs abgehandelte US-Patentschrift 36 37 774):

| Polyglycerin | berechnete Hydroxylzahl |
|---|---|
| Diglycerin bis Pentaglycerin | 1.352 – 1.012 |
| Hexa- bis Decaglycerin | 970 – 888 |
| Undeca- bis Eicosaglycerin | 877 – 825 |
| Heneicosa- bis Triacontaglycerin | 821 – 802 |

**Tabelle**

| Beispiele | Phosphor- und Alkalimetallverbindung | | | | Molverhältnis Alkalimetall zu Phosphor | Reaktionstemperatur °C | Reaktionszeit (Std.) | Hydroxylzahl | Iodfarbzahl |
| | | mol Phosphor | % Phosphor | mol Alkalimetall | | | | | |
|---|---|---|---|---|---|---|---|---|---|
| 1 | $H_3PO_2$ | 0,002 | 0,006 | NaOH | 0,008 | 4 :1 | 250 | 72 | 1360 | <1 |
| 2 | $H_3PO_2$ | 0,006 | 0,02 | KOH | 0,072 | 12 :1 | 220 | 48 | 1320 | 1 |
| 3 | $H_3PO_3$ | 0,016 | 0,05 | NaOH | 0,065 | 4 :1 | 230 | 27 | 1370 | 5 |
| 4 | $H_3PO_2$ | 0,030 | 0,1 | $NaHCO_3$ | 0,064 | 2,1:1 | 240 | 30 | 1360 | <1 |
| 5 | $H_3PO_2$ | 0,065 | 0,2 | NaOH | 0,065 | 1 :1 | 220 | 100 | 1073 | <1 |
| 6 | $H_3PO_2$ | 0,080 | 0,25 | $CH_3ONa$ | 0,400 | 5 :1 | 220 | 22 | 1167 | <1 |
| 7 | $H_3PO_2$ | 0,095 | 0,3 | NaOH | 0,950 | 10 :1 | 220 | 15 | 797 | 5 |
| 8 | $H_3PO_2$ | 0,152 | 0,5 | $Na_2CO_3$ | 0,407 | 2,7:1 | 200 | 30 | 988 | 1 |
| 9 | $H_3PO_2$ | 0,318 | 1,0 | NaOH | 0,705 | 2,2:1 | 190 | 57 | 852 | 1 |
| 10 | $NaH_2PO_2$ | 0,065 | 0,2 | NaOH | 0,130 | *3 :1 | 220 | 46 | 996 | 1 |
| 11 | $NaH_2PO_2$ | 0,048 | 0,15 | $Na_2CO_3$ | 0,120 | *3,5:1 | 210 | 48 | 1292 | 1 |
| 12 | $Na_2HPO_3$ | 0,080 | 0,25 | — | — | 2 :1 | 230 | 55 | 923 | 1 |
| Vergleichsbeispiele | | | | | | | | | |
| 1 | — | — | — | NaOH | 0,400 | — | 220 | 30 | 1345 | 60 |
| 2 | $H_3PO_2$ | 0,075 | 0,24 | — | — | — | 220 | 6 | 1300 | >100 |

* In diesem Wert ist auch das Na aus der P-Verbindung enthalten.

**0 151 755**

**Patentansprüche**

1. Verfahren zur Herstellung von Polyglycerinen, bei dem Glycerin in Gegenwart von Katalysatoren bei einer Temperatur von 190 bis 250 °C unter Entfernung des Reaktionswassers umgesetzt wird, dadurch gekennzeichnet, daß als Katalysator phosphor- und alkalimetallenthaltende Verbindungen eingesetzt werden, so daß 0,005 bis 1 Gew.-%, bezogen auf das Gewicht von Glycerin, Phosphor vorliegt und das Molverhältnis von Alkalimetall zu Phosphor 1 bis 12 : 1 beträgt, wobei die phosphor- und alkalimetallenthaltenden Verbindungen ausgewählt sind aus der Gruppe bestehend aus reduzierenden Phosphorsäuren, Alkalisalzen von reduzierenden Phosphorsäuren, Alkalihydroxiden, Alkalicarbonaten, Alkalibicarbonaten, Alkalialkoholaten und Alkalioxiden.

2. Verfahren nach Anspruch 1, dadurch gekennzeichnet, daß die phosphor- und alkalimetallenthaltenden Verbindungen in einer solchen Menge eingesetzt werden, daß 0,02 bis 0,3 Gew.-% Phosphor vorliegen und das Molverhältnis von Alkalimetall : Phosphor 2 bis 5 : 1 beträgt.

3. Verfahren nach Anspruch 1, dadurch gekennzeichnet, daß als phosphor- und alkalimetallenthaltende Verbindungen Unterphosphorige Säure, Phosphorige Säure, Alkalisalze der Unterphosphorigen Säure, Alkalisalze der Phosphorigen Säure, Alkalihydroxide, Alkalicarbonate, Alkalibicarbonate, Alkalialkoholate, Alkalioxide oder Mischungen davon, eingesetzt werden.

4. Verfahren nach Anspruch 1, dadurch gekennzeichnet, daß als Phosphorverbindungen Unterphosphorige Säure, Phosphorige Säure oder Mischungen davon und als Alkalimetallverbindungen Alkalihydroxide, Alkalicarbonate, Alkalibicarbonate, Alkalialkoholate, Alkalioxide oder Mischungen davon, eingsetzt werden.

5. Verfahren nach Anspruch 1, dadurch gekennzeichnet, daß die Umsetzung bei einer Temperatur von 200 bis 230 °C durchgeführt wird.

6. Verfahren nach Anspruch 5, dadurch gekennzeichnet, daß als Phosphorverbindungen Unterphosphorige Säure, Phosphorige Säure oder Mischungen davon und als Alkalimetallverbindungen Alkalihydroxide, Alkalicarbonate, Alkalibicarbonate oder Mischungen davon in einer solchen Menge eingesetzt werden, daß 0,02 bis 0,3 Gew.-% Phosphor vorliegt und das Molverhältnis von Alkalimetall : Phosphor 2 bis 5 : 1 beträgt.

**Claims**

1. A process for the preparation of a polyglycerol, in which glycerol is reacted in the presence of catalysts at a temperature from 190 to 250°C, the water of reaction being removed, which comprises using compounds containing phosphorus and an alkali metal as the catalyst such that the phosphorus is present in an amount of 0.005 to 1% by weight, based on the weight of glycerol, and the molar ratio of alkali metal to phosphorus is 1 to 12:1, the compounds containing phosphorus and an alkali metal being selected from the group consisting of reducing phosphorus acids, alkali metal salts of reducing phosphorus acids, alkali metal hydroxides, alkali metal carbonates, alkali metal bicarbonates, alkali metal alcoholates and alkali metal oxides.

2. The process as claimed in claim 1, wherein the compounds containing phosphorus and an alkali metal are employed in an amount such that 0.02 to 0.3% by weight of phosphorus is present and the molar ratio of alkali metal phosphorus is 2 to 5:1.

3. The process as claimed in claim 1, wherein hypophosphorous acid, phosphorous acid, alkali metal salts of hypophosphorous acid, alkali metal salts of phosphorous acid, alkali metal hydroxides, alkali metal carbonates, alkali metal bicarbonates, alkali metal alcoholates, alkali metal oxides or mixtures thereof are employed as the compounds containing phosphorus and an alkali metal.

4. The process as claimed in claim 1, wherein hypophosphorous acid, phosphorous acid or a mixture thereof is used as the phosphorus compound and an alkali metal hydroxide, alkali metal carbonate, alkali metal bicarbonate, alkali metal alcoholate, alkali metal oxide or a mixture thereof is used as the alkali metal compound.

5. The process as claimed in claim 1, wherein the reaction is carried out at a temperature from 200 to 230°C.

6. The process as claimed in claim 5, wherein hypophosphorous acid, phosphorous acid or a mixture thereof, is used as the phosphorus compound and an alkali metal hydroxide, alkali metal carbonate, alkali metal bicarbonate or a mixture thereof is used as the alkali metal compound, in an amount such that 0.02 to 0.3% by weight of phosphorus is present and the molar ratio of alkali metal:phosphorus is 2 to 5:1.

**Revendications**

1. Procédé de préparation de polyglycérols selon lequel on fait réagir le glycérol en présence de catalyseurs à une température de 190 à 250°C tout en éliminant l'eau engendrée par le réaction, procédé caractérisé en ce

6

qu'on utilise, comme catalyseurs, des composés contenant du phosphore et un métal alcalin de telle façon qu'il y ait de 0,005 à 1 % en poids de phosphore par rapport au poids du glycérol et que le rapport molaire du métal alcalin au phosphore soit compris entre 1 : 1 et 12 : 1, les composés contenant du phosphore et un métal alcalin étant choisi dans l'ensemble constitué par les acides phosphorés réducteurs, les sels de méteux alcalins des acides phosphorés réducteurs et les hydroxydes, carbonates, hydrogénocarbonates, alcoolates et oxydes de métaux alcalins.

2. Procédé selon la revendication 1 caractérisé en ce que les composés contenant du phosphore et un métal alcalin sont mis en jeu en une quantité telle qu'il y ait de 0,02 à 0,3 % en poids de phosphore et que le rapport molaire du métal alcalin au phosphore soit compris entre 2 : 1 et 5 : 1.

3. Procédé selon le revendication 1 caractérisé en ce qu'on utilise, comme composés contenant du phosphore et un métal alcalin, l'acide hypophosphoreux, l'acide phosphoreux, des sels de métaux alcalins de l'acide hypophosphoreux, des sels de métaux alcalins de l'acide phosphoreux, des hydroxydes de métaux alcalins, des carbonates de métaux alcalins, des hydrogénocarbonates de métaux alcalins, des alcoolates de métaux alcalins, des oxydes de métaux alcalins ou des mélanges de ces composés.

4. Procédé selon la revendication 1 caractérisé en ce qu'on utilise, comme composés du phosphore, l'acide hypophosphoreux, l'acide phosphoreux ou des mélanges de ceux-ce et, comme composés de métaux alcalins, des hydroxydes, carbonates, hydrogénocarbonates, alcoolates ou oxydes de métaux alcalins ou des mélanges de ceux-ci.

5. Procédé selon la revendication 1 caractérisé en ce qu'on effectue la réaction à une température de 200 à 230° C.

6. Procédé selon la revendication 5 caractérisé en ce qu'on utilise, comme composés du phosphore l'acide hypophosphoreux, l'acide phosphoreux ou des mélanges de ceux-ci et, comme composés de métaux alcalins, des hydroxydes, carbonates ou hydrogénocarbonates de métaux alcalins ou des mélanges de ceux-ci, en des quantités telles qu'il y ait de 0,02 à 0,3 % en poids de phosphore et que le rapport molaire du métal alcalin au phosphore soit compris entre 2 : 1 et 5 : 1.